# EUROPÄISCHE PATENTSCHRIFT

(11) **EP 0 486 866 B1**
(45) Veröffentlichungstag und Bekanntmachung des Hinweises auf die Patenterteilung: **22.06.1994**
(21) Anmeldenummer: 91118802.7
(22) Anmeldetag: 05.11.1991
(51) Int. Cl.: C07C 201/16, C07C 205/06

(54) **Verfahren zur Isolierung reiner Dinitrotoluole**
Process for the isolation of purified dinitrotoluenes
Procédé pour l'isolation de dinitrotoluènes purifiés

(30) Priorität: 17.11.1990 DE 4036758
(43) Veröffentlichungstag der Anmeldung: 27.05.1992
(73) Patentinhaber: BAYER AG, 51368 Leverkusen (DE)
(72) Erfinder: Rauchschwalbe, Günther, Dr., W-5090 Leverkusen (DE); Blank, Heinz-Ulrich, Dr., W-5068 Odenthal (DE); Deibele, Ludwig, Dipl.-Ing., W-5000 Köln (DE); Hallenberger, Kaspar, Dipl-Ing., W-5090 Leverkusen (DE); Ruffert, Gerhard, Dipl-Ing., W-5090 Leverkusen (DE)

(56) Entgegenhaltungen:
- DE-A- 3 734 344
- GB-A- 866 421
- US-A- 3 620 928

## Beschreibung

Die Erfindung betrifft ein Verfahren zur Isolierung von stark angereichertem bis sehr reinem 2,4- und 2,6-Dinitrotoluolen (DNT) aus Gemischen, die diese Isomeren enthalten.

Es ist bekannt, daß bei der Nitrierung von Toluol oder von o-Nitrotoluol Isomerengemische erhalten werden, die aus 2,4- und 2,6-DNT im Verhältnis von etwa 65/35 bis zu etwa 85/15 bestehen; außerdem entstehen weitere Isomere (2,5-, 2,3- und 3,4-DNT) in kleinen Mengen von je etwa 0,1 bis 1 %.

Unter der Isolierung der genannten Isomeren im Sinne der Erfindung ist der Erhalt von wenigstens 95 %igen reinen Isomeren, bevorzugt von etwa 99 %ig reinen Isomeren des DNT zu verstehen. Hierbei wird insbesondere das 2,6-DNT in außerordentlich hoher Reinheit von vielfach 99,5 % oder besser erhalten.

Die Trennung der technisch verfügbaren Isomerengemische zur Isolierung angereicherter oder reiner Isomeren ist äußerst schwierig. Aus der Literatur sind Verfahren zur Trennung durch unterschiedliche Adsorption, beispielsweise an Zeolithen, bekannt geworden (US 4.642.397), wobei das an Zeolith selektiv adsorbierte Isomere mit einem anderen Lösungsmittel wieder desorbiert werden muß. Diese Verfahren erfordern demgemäß große Mengen an organischen Lösungsmitteln bzw. Gemischen solcher Lösungsmittel (wie etwa Butanol/Toluol), die aus Gründen des Umweltschutzes mit großem Aufwand abgetrennt, gereinigt und in den Prozeß zurückgeführt werden müssen. Außerdem können nur sehr verdünnte Lösungen auf diese Weise getrennt werden (gemäß Beispiel 1 von US 4 642 397 0,5 g 2,4-DNT und 0,5 g 2,6-DNT in 27,3 g Mesitylen), was die erforderlichen Volumina an organischen Lösungsmitteln weiter in die Höhe treibt. Allein durch diese Beschränkungen ist der beschriebene Prozeß sehr aufwendig und die wirtschaftliche Nutzung im technischen Maßstab behindert.

Weiterhin ist die Trennung von 2,4- und 2,6-DNT durch Schmelzkristallisation bekannt (C.A. 87 (1977), 134 189 y). Die Trennung ist jedoch (vermutlich wegen der geringen Unterschiede der Schmelzpunkte) nicht sehr effizient (Fp. von 2,4-DNT: 71°C; Fp. von 2,6-DNT: 69°C), so daß hohe Reinheiten nur durch wiederholte Kristallisation und mit dem zugehörigen hohen technischen Aufwand erreichbar sind. Außerdem kann hierbei aus der im technischen Maßstab erhältlichen Isomerenmischung nur das 2,4-DNT wirkungsvoll abgetrennt werden; das 2,6-DNT verbleibt angereichert im Rückstand. Die gestellte technische Aufgabe, reines 2,6-DNT herzustellen, kann also mit diesem Verfahren nicht gelöst werden.

Weiterhin sind Versuche bekannt geworden, 2,4- und 2,6-DNT durch Behandlung mit organischen Lösungsmitteln zu trennen.

Im Falle des US-Patentes 3 949 008 (Extraktion mit C5-C8-Alkanen, speziell mit Hexan) konnten dabei nur die Spuren an Nebenisomeren entfernt werden, die Hauptisomeren konnten dagegen nicht separiert werden. Im Falle der Umkristallisation aus Ethanol bzw. C₂HCl₃ (C.A. 95 (1981), 80 328 n) gelingt es nur, das 2,4- DNT in reiner Form herzustellen.

Die Abtrennung von reinem 2,6-DNT aus Isomerengemischen nach US 2 765 348 gelingt nur durch Umkristallisieren aus arbeitshygenisch nicht akzeptablen Lösungsmitteln, wie N,N-Dimethylanilin oder Toluidin. Dabei muß man bei niedrigen Temperaturen (z.B. bei -10°C) abfiltrieren, was einen hohen technischen Aufwand erfordert. Da man das gewünschte Produkt nur in schlechter Ausbeute (z.B. 53 %) und mäßiger Reinheit (z.B. 97 %) erhält, ist dieses Verfahren nicht wirtschaftlich durchzuführen.

Auch durch Auskristallisieren von DNT-Isomerengemischen in Schwefelsäure erhält man in reiner Form nur das 2,4-Isomer (EP 66 202).

Schließlich ist versucht worden, DNT-Gemische mit Reduktionsmitteln zu behandeln, wie Na₂S₂ (US 3 931 347), Na₂SO₃ (JP 45/9169 (1970); zitiert nach C.A. 73 (1970), 14 438 t) oder N₂H₄ (JP 56/142 245 (1981); zitiert nach C.A. 96 (1982), 85 228 k). Dabei werden geringe Mengen unerwünschte Stellungsisomere in wasserlösliche Produkte übergeführt, aber die gewünschten 2,4- und 2,6-Isomeren nicht getrennt.

Die Herstellung von reinem 2,6-DNT ist zwar vorbeschrieben, erfolgt jedoch auf umständlichen Wegen, nämlich
a) durch Diazotieren und Reduzieren von 2,6-Dinitro-4-amino-toluol (Can. J. Chem. 37 (1959), 2073) oder
b) durch Erhitzen von 2,6-Dinitro-phenylessigsäure (Ann. 379 (1911), 152, besondere 181).

Da insbesondere 2,6-DNT ein gesuchtes Zwischenprodukt zur Herstellung von Farbstoffen und Pigmenten, Pharmazeutika, Pflanzenschutzmitteln, Polyurethanen und kosmetischen Produkten ist, war daher anzunehmen, daß eine einfache Herstellmöglichkeit oder Möglichkeit zur Isolierung der obengenannten Produkte bekannt geworden wäre und auch genutzt würde, wenn die Fachwelt solche einfachen Methoden für möglich gehalten hätte.

Es wurde nun überraschend gefunden, daß die Trennung der Hauptisomeren (2,4- und 2,6-DNT) durch Destillation gelingt, in welcher besonders das bisher sehr schwer in reiner Form zugängliche 2,6-DNT in außerordentlich hoher Reinheit (bis zu 99,9 %ig) erhalten werden kann. Eine solche destillative Trennung ist bislang nie beschrieben worden und mußte daher - vor dem Hintergrund der zahlreichen anderen Versuche zur Isomerentrennung - für unmöglich angesehen werden.

Tatsächlich stehen einem solchen Versuch zur destillativen Trennung gravierende Vorurteile entgegen. So wird in DE-OS 37 34 344 zur Verdampfung von Dinitrotoluolen empfohlen, diese durch Zuführen eines heißen inerten Trägergases durchzuführen, da gemäß dieser DE-OS das Arbeiten mit DNT in der Gasphase, zumal im technischen Maßstabe, durch die geringe Flüchtigkeit und die Zersetzlichkeit, die zur Explosion führen kann, belastet ist. Das Trägergas im Verfahren dieser DE-OS liefert dabei die Hauptmenge der zur Verdampfung erforderlichen Energie. Ein solches Vorgehen verringert zwar durch das Vorhandensein des Trägergases den zur Verdampfung bei Normaldruck nötigen Partialdruck des DNT in der Dampfphase und damit seine Temperaturbelastung, doch sind große Mengen an Trägergas erforderlich, da die gebräuchlichen Trägergase, wie Stickstoff und Wasserdampf, nur mit kleinen Mengen an DNT beladen werden können. Diese großen Mengen an Trägergas behindern die bei der Destillation erforderlichen Kondensations- und Stoffaustauschervorgänge und führen zu sehr voluminösen Apparaturen.

Weiterhin müssen grundsätzlich zur Trennung von Verbindungen mit ähnlich liegenden Siedepunkten in einer Destillationskolonne diese Verbindungen in den verschiedenen Trennstufen mehrfach (partiell) verdampft und wieder (partiell) kondensiert werden, wobei sich ein Gleichgewicht einstellt. Zu dieser Gleichgewichtseinstellung ist notwendigerweise einige Zeit erforderlich. Im Widerspruch zu dieser Notwendigkeit der Zeit zur Gleichgewichtseinstellung steht andererseits die Notwendigkeit, die Aufenthaltszeit des DNT in heißen Zonen einer Apparatur möglichst kurz zu halten; folgerichtig werden in der genannten DE-OS für eine (einmalige!) Verdampfung höchstens 120 Sekunden bei 150°C bzw. 2 Sekunden bei 250°C zugelassen. Von dieser Lehre her war nicht zu erwarten, daß eine Kolonnendestillation mit mehrfacher Verdampfung und Kondensation in den einzelnen Trennstufen und mit Aufenthaltszeiten, die weit über den in dieser DE-OS zugelassenen Rahmen hinausgehen, möglich sein könnte.

Weiterhin findet sich in Kirk-Othmer, 3. Auflage, Band II, S. 360 der Hinweis, daß in einem Sumpfphasen-Hydrierverfahren das flüssige DNT aus Sicherheitsgründen bei nicht höher als 100°C gehalten werden muß. Bei dieser Temperatur liegt der Dampfdruck des DNT jedoch sehr niedrig, so daß eine Destillation, vor allem im technischen Maßstab, nicht möglich ist. Diesem Hinweis liegt die Kenntnis zugrunde, daß DNT-Isomerengemische thermisch empfindlich sind und bei entsprechender thermischer Belastung zu heftiger Zersetzung neigen. Dies hat bereits zu schweren Unfällen geführt (Loss Prevention 8 (1974), 117). Die Zersetzung wird offenbar von einer Anzahl von Einflußfaktoren bestimmt und hängt von der Temperatur und der Verweilzeit unter solchen Einflußfaktoren ab; in DTA-Untersuchungen (siehe Loss Prevention, S. 118) wurden bei Heizraten von 2 bis 4°C/min Zersetzungstemperaturen im Bereich von 260 bis 290°C gefunden, aber auch niedrigere Zersetzungstemperaturen sind genannt, beispielsweise 190°C nach 2 Tagen Temperung bzw. 175°C nach 7 Tagen Temperung. Bei den genannten Temperaturen ist DNT bzw. ein Gemisch verschiedener DNT-Isomerer flüssig. Siedepunkte, auch bei vermindertem Druck, sind nicht bekannt.

Man mußte daher davon ausgehen, daß die Fachwelt die Destillation von DNT für nicht möglich hielt, weil sie zu explosiver Zersetzung des DNT führen müßte.

Aus der genannten Literatur ist bekannt, daß DNT innerhalb eines Stabilitätskriteriums gehandhabt werden kann, das aus Verweilzeit und Temperatur definiert ist, beispielsweise unterhalb von 23 Tagen bei 150°C, unterhalb von 7 Tagen bei 175°C bzw. unterhalb von 2 Tagen bei 190°C, bevor heftige Zersetzung eintritt (Loss Prevention, S. 120). In eigenen Versuchen erwiesen sich die genannten Werte jedoch als schlecht reproduzierbar. Vor allem mit technisch verfügbaren und daher verunreinigten Einsatzwaren wurden immer wieder Wertepaare aus Temperatur und Temperungszeit gefunden, die deutlich geringere thermische Stabilitäten solcher DNT-Gemische auswiesen. Das machte bisher eine technische Destillation von technischen DNT-Gemischen aus Sicherheitsgründen unmöglich.

Es wurde ein Verfahren zur Isolierung von reinem 2,4- und 2,6-Dinitrotoluol gefunden, das dadurch gekennzeichnet ist, daß man ein diese Isomeren enthaltendes Gemisch unter Ausschluß von reduzierenden Bedingungen unter einem Druck von 0,5 bis 20 mbar und bei einer Temperatur von 80 bis 200°C destilliert.

Das erfindungsgemäße Verfahren ergibt ein sehr stark angereichertes 2,4-Isomer (wenigstens 95 %ig) als Sumpfprodukt und ein sehr reines 2,6- Isomer (mindestens 99 %ig) als Kopfprodukt. Besonders überraschend ist, daß die beiden sehr ähnlichen Isomeren mit geringen Rücklaufverhältnissen destillativ getrennt werden können, wobei sich geringe Verweilzeiten in der Kolonne ergeben, die trotz der Berücksichtigung der Sicherheitsaspekte eine effiziente Trennung der beiden genannten Isomeren erlauben.

Es ist ein wichtiges Kennzeichen des erfindungsgemäßen Verfahrens, daß die destillative Trennung so durchgeführt werden muß, daß das zu trennende Isomerengemisch unter Ausschluß von reduzierenden Bedingungen gehandhabt wird. Reduzierende Bedingungen, die erfindungsgemäß ausgeschlossen werden müssen, treten beispielsweise auf
a) durch die Gegenwart von reduzierten Nebenprodukten, die beispielsweise von einer thermischen Belastung herrühren oder von einer Behandlung mit reduzierenden Reagenzien zur Entfernung bestimmter Isomeren (siehe obengenannte Literatur) oder zur Entfernung von verfärbenden Oxidationsprodukten, die bei der thermischen Nitrierung von Toluol oder Nitrotoluol entstehen; oder
b) durch die Anwesenheit von technisch bedingten Säurespuren in Verbindung mit reduzierend wirkenden Apparateteilen (beispielsweise Eisen);
c) auch durch den Kontakt mit Alkalien, der bereits bei etwa 100°C die Bildung reduzierter Verbindungen auslösen kann , was beispielsweise aus dem strukturell verwandten Fall von 2-Nitrotoluol-4-sulfonsäure bekannt ist (DE-PS 138 188 (1900)). Dies steht in Übereinstimmung mit der Beobachtung, daß ein technisches DNT-Gemisch, das 2,4- und 2,6-DNT im Verhältnis 65/35 enthält, in der Differentialthermoanalyse (DTA) bei einer Heizrate von 0,05°C/min eine stark exotherme Zersetzung erst ab 215°C zeigt, während unter gleichen Bedingungen eine starke Exothermie bereits ab 130°C beobachtet wird, wenn das DNT kleine Mengen von Toluylendiamin enthält. Diese zuletzt geschilderten Bedingungen liegen vor, wenn DNT in der Sumpfphasenhydrierung in Toluylendiamin umgewandelt wird (vgl. die genannte Literatur Kirk-Othmer). Die, wie oben geschildert, bei der Sumpfphasenhydrierung einzuhaltende obere Temperaturgrenze von 100°C ist durch die Anwesenheit der bereits reduzierten Stoffe bedingt, die die Stabilität des DNT herabsetzen; dieser Zusammenhang war bisher nicht erkannt worden, wurde jedoch im Zusammenhang mit der vorliegenden Erfindung gefunden und untersucht.

Der Ausschluß von reduzierenden Bedingungen kann durch verschiedene Maßnahmen erreicht werden. So kann die Anwesenheit reduzierender Agenzien von vornherein ausgeschlossen werden. Weiterhin können vorhandene Isomerengemische durch geeignete Maßnahmen von reduzierenden Beimischungen befreit werden. Hierzu wird beispielsweise die Einsatzware zunächst mit Säure gewaschen, um reduzierte Nebenprodukte (hauptsächlich Amine) zu entfernen. Sodann wird mit wenig Alkali gewaschen, um Säurereste zu entfernen, die zu Korrosion und Reduktion führen würde. Danach wird mit viel Wasser gewaschen, um Alkalireste mit Sicherheit zu entfernen, die beim folgenden Erhitzen die Bildung von reduzierten Verbindungen auslösen könnte. Des weiteren wird ein technisches DNT-Gemisch in korrosionsfesten Apparaturen gehandhabt, um das Entstehen reduzierter Verbindungen von vornherein auszuschließen. Als korrosionsfeste Werkstoffe seien beispielsweise rostfreier Stahl, emaillierter Stahl, Glas oder Hastelloy genannt. Die Verwendung anderer korrosionsfester Materialien, wie sie dem Fachmann bekannt sind, ist ebenfalls möglich.

Es hat sich weiterhin als günstig erwiesen, jede zur Destillation vorgesehene Charge analytisch auf Säure- bzw. Alkalireste sowie auf ihre thermische Stabilität zu überprüfen.

Das DNT-Gemisch wird bevorzugt schonend verdampft, wobei die Verwendung eines Fallfilmverdampfers besonders vorteilhaft ist, da in diesem Apparat bei gutem Wärmeübergang eine nur geringe Verweilzeit im Verdampfer erzielt werden kann. Ferner ist es vorteilhaft, als Heizmittel in einem Verdampfer Wärmeträgerflüssigkeiten einzusetzen, deren Siedepunkt unterhalb der Zersetzungstemperatur des DNT-Gemisches liegt. Solche Wärmeträgerflüssigkeiten können beispielsweise Dichlorbenzol, besonders bevorzugt m-Dichlorbenzol, sein.

Das verdampfte DNT-Gemisch wird sodann in den mittleren Bereich einer kontinuierlich arbeitende Destillationskolonne geleitet, die aus Abtreiber- und Verstärkerteil besteht und mit Einbauten oder Füllkörpern mit möglichst geringem Druckverlust bestückt ist. Man trennt bei einem Rückflußverhältnis von Rückfluß:Entnahme = 1-10:1, bevorzugt 2-5:1, besonders bevorzugt 2,5-3,5:1. Der Druck in der Kolonne liegt erfindungsgemäß bei 0,5 bis 20 mbar, bevorzugt bei 1 bis 15 mbar. Die Temperatur liegt erfindungsgemäß in der Destillationskolonne bei 80 bis 200°C in Abhängigkeit vom Druck, bevorzugt bei 100 bis 180°C, besonders bevorzugt bei 90 bis 170°C.

Zur Steuerung dient bevorzugt die Messung der Sumpftemperatur, wo die Temperatur am höchsten und damit am dichtesten an den sicherheitstechnischen Verfahrensgrenzen liegt.

Im Rahmen der genannten Bedingungen, insbesondere durch Einstellung der Rückflußverhältnisse wird die Isolierung in der Destillationskolonne so gesteuert, daß die DNT-Isomeren in Reinheiten von etwa 90 % bis 99,99 % erhalten werden.

Das Destillat (2,6-DNT) wird kondensiert und flüssig aus der Apparatur entnommen.

Die so erhaltenen reinen DNT sind geeignet als Zwischenprodukte, und werden hierzu im allgemeinen durch katalytische Hydrierung zu reinen Diaminotoluolen weiterverarbeitet, die ihrerseits die obengenannte technische Verwendung finden.

Das erfindungsgemäße Verfahren zur destillativen Isolierung der DNT-Isomeren ist besonders überraschend, wenn man die eigene Erkenntnis heranzieht, daß die strukturell sehr ähnlichen Diaminotoluol-Gemische, die durch Reduktion aus den DNT-Gemischen erhalten werden können, in der geschilderten Art nicht getrennt werden können.

### Beispiel 1

In einer kontinuierlich betriebenen Destillationskolonne (500 mm Durchmesser, 1,80 m im Verstärkerteil, 1 m Höhe im Abtreiberteil, mit Sulzer-BX-Backung, 6-7 Trennstufen pro m, aus rostfreiem Stahl) wurde bei einem Zulauf von 100 kg Dinitrotoluol(DNT)-Gemisch pro Stunde (65 % 2,4-, 35 % 2,6-Isomer) bei einem Rücklaufverhältnis von 3:1, einer Sumpftemperatur von 140 bis 145°C, einer Kopftemperatur von 94°C, einem Druck im Sumpf von 4,5 mbar und einem Druck am Kopf von 1 mbar destilliert. Dabei destillierte am Kopf des Verstärkereils das 2,6-DNT in 99 %iger Reinheit ab, während das 2,4-DNT in ca. 95-97 %iger Reinheit dem Sumpf entnommen wurde.

### Beispiel 2

In einer kontinuierlich betriebenen Destillationskolonne (500 mm Durchmesser, 2,5 m Höhe im Verstärkerteil, 1,5 m Höhe im Abtreiberteil, mit Sulzer-BX-Packung, aus korrosionsfreiem Stahl) wurde bei einem Zulauf von 100 kg/h DNT-Gemisch wie in Beispiel 1 und einem Rücklaufverhältnis von 3:1, einer Sumpftemperatur von 170°C, einer Kopftemperatur von 132°C, einem Druck im Sumpf von 13 mbar und einem Druck am Kopf von 5 mbar destilliert. Dabei destillierte am Kopf der Kolonne das 2,6-DNT in 99,9 %iger Reinheit ab, während das 2,4-DNT in 97-98 %iger Reinheit dem Sumpf entnommen wurde.

## Patentansprüche

1. Verfahren zur Isolierung von reinem 2,4- und 2,6-Dinitrotoluol, dadurch gekennzeichnet, daß man ein diese Isomeren enthaltendes Gemisch unter Ausschluß von reduzierenden Bedingungen unter einem Druck von 0,5 bis 20 mbar und bei einer Temperatur von 80 bis 200°C destilliert.

2. Verfahren nach Anspruch 1, dadurch gekennzeichnet, daß der Druck bei 1 bis 15 mbar liegt.

3. Verfahren nach Anspruch 1, dadurch gekennzeichnet, daß die Temperatur 100 bis 180°C, bevorzugt 90 bis 170°C beträgt.

4. Verfahren nach Anspruch 1, dadurch gekennzeichnet, daß die zu steuernde Temperatur die des Kolonnensumpfes ist.

5. Verfahren nach Anspruch 1, dadurch gekennzeichnet, daß das zu trennende Dinitrotoluol-Gemisch in einem Kurzzeitverdampfer, bevorzugt in einem Fallfilmverdampfer, verdampft wird und im mittleren Bereich der Destillationskolonne dampfförmig eingespeist wird.

6. Verfahren nach Anspruch 5, dadurch gekennzeichnet, daß der Kurzzeitverdampfer mit Hilfe einer Wärmeträgerflüssigkeit beheizt wird, deren Siedetemperatur unterhalb der Zersetzungstemperatur des Dinitrotoluolgemisches liegt.

7. Verfahren nach Anspruch 1, dadurch gekennzeichnet, daß Gemische eingesetzt werden, die das 2,4- und das 2,6-Isomere im Verhältnis 65:35 bis 85:15 enthalten.

8. Verfahren nach Anspruch 1, dadurch gekenzeichnet, daß Rückflußverhältnisse von Rückfluß : Entnahme = 1-10:1, bevorzugt 2-5:1, besonders bevorzugt 2,5-3,5:1 eingestellt werden.

9. Verfahren nach Anspruch 1, dadurch gekennzeichnet, daß das zu trennende Dinitrotoluol-Gemisch a) mit Säure, b) mit Alkali bis zur Entfernung der Säure und c) mit Wasser bis zur Entfernung des Alkali behandelt wird.

10. Verfahren nach Anspruch 1, dadurch gekennzeichnet, daß die Destillation in korrosionsfesten Apparaturen vorgenommen wird.

## Claims

1. Process for the isolation of pure 2,4- and 2,6-dinitrotoluene, characterized in that a mixture containing these isomers is distilled under a pressure of 0.5 to 20 mbar and at a temperature of 80 to 200°C with exclusion of reducing conditions.

2. Process according to Claim 1, characterized in that the pressure is 1 to 15 mbar.

3. Process according to Claim 1, characterized in that the temperature is 100 to 180°C, preferably 90 to 170°C.

4. Process according to Claim 1, characterized in that the temperature to be controlled is that of the bottom of the column.

5. Process according to Claim 1, characterized in that the dinitrotoluene mixture to be separated is evaporated in a short-time evaporator, preferably in a falling film evaporator, and is fed into the central region of the distillation column in the form of a vapour.

6. Process according to Claim 5, characterized in that the short-term evaporator is heated with the aid of a heat transfer liquid, the boiling point of which is below the decomposition point of the dinitrotoluene mixture.

7. Process according to Claim 1, characterized in that mixtures which contain the 2,4- and 2,6-isomer in a ratio of 65:35 to 85:15 are employed.

8. Process according to Claim 1, characterized in that reflux ratios of reflux:withdrawal = 1-10:1, preferably 2-5:1, particularly preferably 2.5-3.5:1, are established.

9. Process according to Claim 1, characterized in that the dinitrotoluene mixture to be separated is treated a) with acid, b) with alkali until the acid has been removed and c) with water until the alkali has been removed.

10. Process according to Claim 1, characterized in that the distillation is carried out in corrosion-resistant apparatuses.

## Revendications

1. Procédé pour isoler du 2,4-dinitrotoluène et du 2,6-dinitrotoluène purs, caractérisé en ce qu'on distille un mélange contenant ces isomères, en excluant des conditions réductrices, sous une pression de 0,5 à 20 mbars et à une température de 80 à 200°C.

2. Procédé suivant la revendication 1, caractérisé en ce que la pression se situe entre 1 et 15 mbars.

3. Procédé suivant la revendication 1, caractérisé en ce que la température se situe entre 100 et 180°C, de préférence entre 90 et 170°C.

4. Procédé suivant la revendication 1, caractérisé en ce que la température que l'on doit régler est celle du bas de la colonne.

5. Procédé suivant la revendication 1, caractérisé en ce que le mélange de dinitrotoluènes à séparer est vaporisé dans un évaporateur à courte durée d'action, de préférence dans un évaporateur à film tombant et il est injecté sous forme de vapeur dans la région moyenne de la colonne de distillation.

6. Procédé suivant la revendication 5, caractérisé en ce que l'évaporateur à courte durée d'action est chauffé à l'aide d'un fluide caloporteur dont la température d'ébullition est en-dessous de la température de décomposition du mélange de dinitrotoluènes.

7. Procédé suivant la revendication 1, caractérisé en ce qu'on utilise des mélanges qui contiennent les isomères 2,4 et 2,6 dans un rapport de 65:35 à 85:15.

8. Procédé suivant la revendication 1, caractérisé en ce qu'on règle des rapports reflux : soutirage de 1-10:1, de préférence de 2-5:1, notamment de 2,5-3,5:1.

9. Procédé suivant la revendication 1, caractérisé en ce qu'on traite le mélange de dinitrotoluènes à séparer a) avec un acide, b) avec un alcali jusqu'à l'élimination de l'acide et c) avec de l'eau jusqu'à l'élimination de l'alcali.

10. Procédé suivant la revendication 1, caractérisé en ce qu'on effectue la distillation dans des appareils résistants à la corrosion.
